(19) Europäisches Patentamt
European Patent Office
Office européen des brevets

(11) EP 3 569 691 A1

(12) **EUROPEAN PATENT APPLICATION**
published in accordance with Art. 153(4) EPC

(43) Date of publication:
20.11.2019 Bulletin 2019/47

(21) Application number: 17890991.7

(22) Date of filing: 27.12.2017

(51) Int Cl.:
*C12N 5/071* (2010.01)      *A61K 35/30* (2015.01)
*A61L 27/38* (2006.01)      *A61P 27/02* (2006.01)
*C12N 5/10* (2006.01)      *A61K 35/545* (2015.01)

(86) International application number:
PCT/JP2017/047031

(87) International publication number:
WO 2018/131491 (19.07.2018 Gazette 2018/29)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(30) Priority: 13.01.2017 JP 2017004419

(71) Applicant: Osaka University
Suita-shi, Osaka 565-0871 (JP)

(72) Inventors:
• NISHIDA, Kohji
Suita-shi
Osaka 565-0871 (JP)
• HAYASHI, Ryuhei
Suita-shi
Osaka 565-0871 (JP)
• SHIBATA, Shun
Suita-shi
Osaka 565-0871 (JP)

(74) Representative: Witte, Weller & Partner
Patentanwälte mbB
Postfach 10 54 62
70047 Stuttgart (DE)

(54) **METHOD FOR PRODUCING CORNEAL EPITHELIAL CELL MASS**

(57) The present invention provides a method for producing a corneal epithelial cell population for the fabrication of corneal epithelial cell sheets for transplantation, the method comprising the steps of:
(1) preparing a cell population containing corneal epithelial cells;
(2) subjecting the cell population prepared in step (1) to magnetic-activated cell sorting and collecting CD200-negative/SSEA-4-positive cells; and
(3) bringing the cells collected in step (2) into contact with a laminin selected from the group consisting of a laminin having an $\alpha 3$ chain, a laminin having an $\alpha 4$ chain and a laminin having an $\alpha 5$ chain, or an E8 fragment thereof, and collecting cells adherent thereto.

Printed by Jouve, 75001 PARIS (FR)

**Description**

TECHNICAL FIELD

The present invention relates to a method for producing a corneal epithelial cell population. In particular, the present invention relates to a method for producing a high-purity corneal epithelial cell population for use in fabricating corneal epithelial cell sheets for transplantation.

BACKGROUND ART

Corneal epithelial cells are located on the outermost ocular surface and serve important functions, such as barrier function against the outer environment. A group of intractable eye diseases including Stevens-Johnson syndrome, ocular cicatricial pemphigoid and alkali burn is known as limbal stem cell deficiency. In limbal stem cell deficiency, corneal epithelial stem cells, which are present in the limbus, are irreversibly dysfunctional or lost due to trauma, chronic inflammatory responses such as autoimmunity, etc. As a result, conjunctival epithelium migrates onto the central part of the corneal surface, resulting in a loss of transparency of the cornea, i.e. corneal opacity, leading to a severe decline in vision. A conventional treatment for restoring the vision of patients with serious corneal diseases such as limbal stem cell deficiency is corneal transplantation. In particular, for patients with binocular disease (disease develops in both eyes), allogeneic corneal transplantation is the only current treatment because patients do not have their own healthy corneas . However, allogeneic corneal transplantation suffers from the shortage of compatible cornea donors and the risk of rejection of donor's cornea. Therefore, the development of therapies using an autologous cell source is desired.
In order to provide a novel therapy for serious corneal diseases, such as limbal stem cell deficiency, the present inventors developed a methodology for fabricating a corneal epithelial cell sheet from iPS cells and examined the efficacy of the cell sheet in an animal model (Non Patent Literature 1) . The iPS cell-derived cells obtained by the differentiation method described in Non Patent Literature 1 compose a population of various periocular cells. For this reason, the use of such a cell population for the fabrication of corneal epithelial cell sheets requires purification of corneal epithelial cells. Non Patent Literature 1 describes purification of corneal epithelial cells by FACS sorting using antibodies against cell surface markers specific to corneal epithelial cells.
FACS sorting is a technique that allows isolation of specific cells based on cell surface antigen expression which is examined by laser irradiation to antibody-stained cells passed in a stream of fluid. This technique, however, has difficulty in ensuring the sterility of the entire flow path through which cells pass, and also has the risk of cell contamination. In addition, cells collected after FACS sorting are heavily damaged. Moreover, the maintenance of the apparatus used for FACS sorting requires special knowledge and skills. For these reasons, FACS sorting is not necessarily an ideal technique in the setting to prepare a large amount of cells used for the fabrication of corneal epithelial cell sheets for transplantation. That is, the methodology of Non Patent Literature 1 still has problems to be solved for practical and industrial use.

CITATION LIST

Non Patent Literature

Non Patent Literature 1:
Hayashi et al., Nature. 2016 Mar 17;531(7594):376-80. doi: 10.1038/nature17000. Epub 2016 Mar 9.

SUMMARY OF INVENTION

TECHNICAL PROBLEM

An object of the present invention is to provide a method for mass producing a high-purity, safe and less damaged corneal epithelial cell population from a cell population containing corneal epithelial cells in a short time by a simple procedure without using FACS sorting, which corneal epithelial cell population can be used for the fabrication of corneal epithelial cell sheets for transplantation.

SOLUTION TO PROBLEM

In order to achieve the above-mentioned object, the present invention includes the following.

[1] A method for producing a corneal epithelial cell population for fabrication of corneal epithelial cell sheets for transplantation, the method comprising the steps of:

(1) preparing a cell population containing corneal epithelial cells;
(2) subjecting the cell population prepared in step (1) to magnetic-activated cell sorting and collecting CD200-negative/SSEA-4-positive cells; and
(3) bringing the cells collected in step (2) into contact with a laminin selected from the group consisting of a laminin having an $\alpha$3 chain, a laminin having an $\alpha$4 chain and a laminin having an $\alpha$5 chain, or an E8 fragment thereof, and collecting cells adherent thereto.

[2] The method according to the above [1], wherein step (3) comprises the substeps of:

(3-1) bringing the cells collected in step (2) into contact with a laminin having an $\alpha$2 chain or an E8 fragment thereof, and collecting non-adherent cells; and
(3-2) bringing the collected non-adherent cells into contact with a laminin selected from the group consisting of a laminin having a laminin having an $\alpha$3 chain, a laminin having an $\alpha$4 chain and a laminin having an $\alpha$5 chain, or an E8 fragment thereof, and collecting cells adherent thereto.

[3] The method according to the above [1] or [2], wherein the cell population containing corneal epithelial cells is a periocular cell population derived by induced differentiation of pluripotent stem cells.
[4] The method according to any of the above [1] to [3], wherein the proportion of PAX6-positive/p63-positive cells in the corneal epithelial cell population for fabrication of corneal epithelial cell sheets for transplantation is 50% or more.
[5] The method according to any of the above [1] to [4], wherein the laminin selected from the group consisting of a laminin having an $\alpha$3 chain, a laminin having an $\alpha$4 chain and a laminin having an $\alpha$5 chain, or an E8 fragment thereof is a laminin $\alpha$3$\beta$3$\gamma$2 E8 fragment, a laminin $\alpha$4$\beta$1$\gamma$1 E8 fragment or a laminin $\alpha$5$\beta$1$\gamma$1 E8 fragment.
[6] The method according to the above [2], wherein the laminin having an $\alpha$2 chain or an E8 fragment thereof is a laminin $\alpha$2$\beta$1$\gamma$1 E8 fragment.
[7] A method for fabricating a corneal epithelial cell sheet for transplantation, the method comprising the step of culturing the corneal epithelial cell population produced by the method according to any of the above [1] to [6].

ADVANTAGEOUS EFFECTS OF INVENTION

According to the present invention, a high-purity, safe and less damaged corneal epithelial cell population that can be used for the fabrication of corneal epithelial cell sheets for transplantation can be mass produced from a cell population containing corneal epithelial cells in a short time by a simple procedure without using FACS sorting.

BRIEF DESCRIPTION OF DRAWINGS

Fig. 1 shows the results of separation of CD200-negative/SSEA-4-positive/ITGB4-positive cells by FACS sorting from periocular cells differentiated from iPS cells. Fig. 1A shows the area of CD200-negative cells, and Fig. 1B shows the area of CD200-negative/SSEA-4-positive/ITGB4-positive cells.
Fig. 2 shows images of FACS-sorted CD200-negative/SSEA-4-positive/ITGB4-positive cells after 3-week culture. Fig. 2A shows the results of phase-contrast microscopic observation of the cells, and Fig. 2B shows the results of the analysis of the proteins expressed in the resulting cell sheet.
Fig. 3 shows the results of FACS analysis of the expression of CD200, SSEA-4 and ITGB4 in periocular cells differentiated from iPS cells; cells after depletion of CD200-positive cells from the periocular cells by MACS; and SSEA-4-positive cells isolated by MACS from the CD200-positive cell-depleted cell population.
Fig. 4 graphically illustrates the results of FACS analysis shown in Fig. 3.
Fig. 5 shows the results of FACS analysis of the expression of CD200, SSEA-4 and ITGB4 in non-adherent cells collected from plates coated with different laminin E8 fragments on which periocular cells differentiated from iPS cells had been seeded.
Fig. 6 shows the percentage of corneal epithelial cells in non-adherent cells collected from laminin 211E8 fragment-coated plates on which periocular cells differentiated from iPS cells had been seeded.
Fig. 7 shows the expression of marker genes for corneal epithelial cells and marker genes for non-corneal epithelial cells in adherent cells collected from laminin 211E8 fragment-coated plates on which periocular cells differentiated from iPS cells had been seeded.
Fig. 8 shows the expression of marker genes for corneal epithelial cells and marker genes for non-corneal epithelial cells in the cell populations at different stages in the process of producing a corneal epithelial cell population by the production method of the present invention from periocular cells differentiated from iPS cells.

Fig. 9 shows the results of phase-contrast microscopic observation of the corneal epithelial cell population that had been produced by the production method of the present invention and subsequently cultured on a laminin 511E8 fragment until grown to confluency for 3 weeks.

Fig. 10 shows the results of the analysis of the proteins expressed in the cell sheet obtained by producing a corneal epithelial cell population by the production method of the present invention and subsequently culturing the cell population on a laminin 511E8 fragment for 3 weeks.

Fig. 11 shows the results of FACS analysis of K14-positive cells and K12-positive cells in the corneal epithelial cell population produced by the production method of the present invention and in FACS-sorted CD200-negative/SSEA-4-positive/ITGB4-positive cells. The FACS analysis was done after the corneal epithelial cell population and the FACS-sorted cells were subjected to 3-week culture on a laminin 511E8 fragment, detachment and dissociation.

Fig. 12 shows the comparison of the morphology between cell sheets formed from the corneal epithelial cell populations (MLS (322), MLS (511)) produced by the production method of the present invention and cell sheets formed form cell populations produced by different methods.

Fig. 13 shows phase-contrast microscopic images and fluorescence microscopic images of the corneal epithelial cells purified with the use of different kinds of laminins E8s from periocular cells differentiated from epithelial stem cell marker p63 knock-in iPS cells in which p63 and EGFP are co-expressed.

Fig. 14 shows the calculated percentage of p63-positive cells in the corneal epithelial cells purified with the use of different kinds of laminins E8s from periocular cells differentiated from epithelial stem cell marker p63 knock-in iPS cells in which p63 and EGFP are co-expressed.

DESCRIPTION OF EMBODIMENTS

According to Non Patent Literature 1, in order to purify corneal epithelial cells from a periocular cell population derived by induced differentiation of iPS cells, the present inventors used FACS to first collect cells not expressing TRA-1-60 (TRA-1-60-negative cells), which is a marker expressed on undifferentiated iPS cells. Then, from the collected cells, cells positive for SSEA-4 and integrin β4 (hereinafter also referred to as "ITGB4"), which are markers expressed on corneal epithelial cells, were collected by FACS. The thus-collected cell population was used as a corneal epithelial cell population. However, during the culture of such a TRA-1-60-negative/SSEA-4-positive/ITGB4-positive cell population, colony formation of contaminant cells, i.e., cells different from corneal epithelial cells was sometimes observed, indicating an insufficient purity of corneal epithelial cells. To address this problem, the present inventors comprehensively analyzed the genes expressed in corneal epithelial cell colonies and contaminant cell colonies formed during the culture of the TRA-1-60-negative/SSEA-4-positive/ITGB4-positive cell population, and as a result, identified CD200 as a gene highly expressed in the contaminant cell colonies. In the situation where there were no reports that CD200 is expressed in pluripotent stem cells or that CD200 is not expressed in corneal epithelial cells, the present inventors first found that CD200 is a very excellent selection marker with which the depletion of more diverse contaminant cells can be achieved as compared with the known selection marker TRA-1-60. Therefore, the "corneal epithelial cells" of the present invention are synonymous with "CD200-negative/SSEA-4-positive/ITGB4-positive cells".

The present invention provides a method for producing a corneal epithelial cell population for the fabrication of corneal epithelial cell sheets for transplantation (hereinafter referred to as the "production method of the present invention"). The production method of the present invention comprises the steps of:

(1) preparing a cell population containing corneal epithelial cells;
(2) subjecting the cell population prepared in step (1) to magnetic-activated cell sorting and collecting CD200-negative/SSEA-4-positive cells; and
(3) bringing the cells collected in step (2) into contact with a laminin selected from the group consisting of a laminin having an α3 chain, a laminin having an α4 chain and a laminin having an α5 chain, or an E8 fragment thereof, and collecting cells adherent thereto.

In step (1), a cell population containing corneal epithelial cells is prepared. The cell population containing corneal epithelial cells can be prepared by induced differentiation of pluripotent stem cells. Alternatively, the cell population containing corneal epithelial cells can be prepared from a tissue containing a cornea, such as a donor cornea. The pluripotent stem cells are stem cells which have pluripotency, i.e., the ability to differentiate into any type of cells present in a living body, and also have proliferative capacity. Examples of the stem cells include embryonic stem cells (ES cells), embryonic stem cells from a cloned embryo obtained by nuclear transfer (ntES cells), spermatogenic stem cells (GS cells), embryonic germ cells (EG cells), induced pluripotent stem cells (iPS cells), and pluripotent cells from cultured fibroblasts or myeloid stem cells (Muse cells). Preferred are ES cells, ntES cells and iPS cells, and more preferred are iPS cells. The pluripotent stem cells are preferably pluripotent stem cells of mammals. The mammal is not particularly limited, and examples include humans, mice, rats, cattle and pigs. Particularly preferred are humans. With the use of human pluripotent stem cells, a corneal epithelial cell population that is safe and compatible for use in human regenerative medicine can be obtained.

The method for preparing the cell population containing corneal epithelial cells by induced differentiation of pluripotent stem cells is not particularly limited and can be selected as appropriate from known methods. For example, the method developed by the present inventors to induce pluripotent stem cells to differentiate into periocular cells (Non Patent Literature 1) can preferably be used. This differentiation method comprises culturing maintained human iPS cells in a medium containing human keratinocyte growth factor (KGF) and human fibroblast growth factor-2 (FGF-2). However, in this differentiation method, which is described in Non Patent Literature 1, FGF-2 is not essential for the induction of corneal epithelial cells. Even when FGF-2 is excluded from the medium described in Non Patent Literature 1, a cell population containing corneal epithelial cells can be prepared from human iPS cells by induced differentiation (see Reference Example 1) . In addition to these methods, other differentiation methods can also be used to prepare a cell population containing corneal epithelial cells from pluripotent stem cells, and examples are described in the following references (a) to (e).

(a) Shalom-Feuerstein, R., Serror, L., De La Forest Divonne, S., Petit, I., Aberdam, E., Camargo, L., Damour, O., Vigouroux, C., Solomon, A., Gaggioli, C., et al. (2012) . Pluripotent stem cell model reveals essential roles for miR-450b-5p and miR-184 in embryonic corneal lineage specification. Stem Cells 30, 898-909.
(b) Hewitt, K.J., Shamis, Y., Carlson, M.W., Aberdam, E., Aberdam, D., and Garlick, J.A. (2009). Three-dimensional epithelial tissues generated from human embryonic stem cells. Tissue Eng. Part A 15, 3417-3426.
(c) Hayashi, R., Ishikawa, Y., Ito, M., Kageyama, T., Takashiba, K., Fujioka, T., Tsujikawa, M., Miyoshi, H., Yamato, M., Nakamura, Y., and Nishida, K. (2012). Generation of corneal epithelial cells from induced pluripotent stem cells derived from human dermal fibroblast and corneal limbal epithelium. PLoS ONE 7, e45435.
(d) Hanson, C., Hardarson, T., Ellerstrom, C., Nordberg, M., Caisander, G., Rao, M., Hyllner, J., and Stenevi, U. (2013). Transplantation of human embryonic stem cells onto a partially wounded human cornea in vitro. Acta Ophthalmol. (Copenh.) 91, 127-130.
(e) Ahmad, S., Stewart, R., Yung, S., Kolli, S., Armstrong, L., Stojkovic, M., Figueiredo, F., and Lako, M. (2007). Differentiation of human embryonic stem cells into corneal epithelial-like cells by in vitro replication of the corneal epithelial stem cell niche. Stem Cells 25, 1145-1155.

The method for preparing the cell population containing corneal epithelial cells from a tissue containing a cornea, such as a donor cornea, is not particularly limited and can be selected as appropriate from known methods. For example, corneal epithelial cells may be scraped from the sclerocorneal tissue of a donor cornea with forceps or the like under a stereomicroscope and cultured to prepare a cell population containing corneal epithelial cells (Hayashi R, et al., Tissue Eng Part C Methods. 16(4):553-560, 2010.)

The cell population containing corneal epithelial cells used in the production method of the present invention is preferably a cell population prepared by the method described in Non Patent Literature 1, specifically a cell population prepared by induced differentiation of human iPS cells into periocular cells.

In step (2), the cell population prepared in step (1) is subjected to magnetic-activated cell sorting (hereinafter also referred to as "MACS"), and CD200-negative/SSEA-4-positive cells are collected. Step (2) can be performed using a commercial magnetic-activated cell sorter. Usually, depletion of CD200-positive cells is followed by collection of SSEA-4-positive cells, but any alternative procedure may be employed without limitations. For the depletion of CD200-positive cells using MACS, for example, the cell population prepared in step (1) is stained with an anti-CD200 antibody, brought into contact with magnetic microbeads that bind to the anti-CD200 antibody (e.g., anti-immunoglobulin antibody-immobilized magnetic microbeads), and applied to a magnetic column of a magnetic-activated cell sorter, and the cells passed through the column are collected as a CD200-positive cell-depleted cell population (CD200-negative cell population) . The anti-CD200 antibody may be in a labeled form. In the case where a labeled anti-CD200 antibody is used, magnetic microbeads that bind to the labeling substance can be used. In the case where anti-CD200 antibody-immobilized magnetic microbeads can be used, the step of cell staining with an anti-CD200 antibody can be skipped.

Next, for collection of SSEA-4-positive cells, for example, the collected CD200-negative cell population is stained with an anti-SSEA4 antibody, brought into contact with magnetic microbeads that bind to the anti-SSEA4 antibody (e.g., anti-immunoglobulin antibody-immobilized magnetic microbeads), and applied to a magnetic column of a magnetic-activated cell sorter, and the cells retained in the column are collected as CD200-negative/SSEA-4-positive cells. For the collection of the cells retained in the column, for example, the column is departed from the magnetic field, and the cells are eluted with an appropriate buffer. The anti-SSEA4 antibody may be in a labeled form. In the case where a labeled anti-SSEA4 antibody is used, magnetic microbeads that bind to the labeling substance can be used. In the case where anti-SSEA4 antibody-immobilized magnetic microbeads can be used, the step of cell staining with an anti-SSEA4 antibody can be skipped.

In step (3), the cells collected in step (2) are brought into contact with a laminin selected from the group consisting of a laminin having an α3 chain, a laminin having an α4 chain and a laminin having an α5 chain, or an E8 fragment thereof, and cells adherent thereto are collected. In another embodiment, firstly, the cells collected in step (2) are brought into contact with a laminin having an α2 chain or an E8 fragment thereof, and non-adherent cells are collected. Then, the collected non-adherent cells are brought into contact with a laminin selected from the group consisting of a laminin having a laminin having an α3 chain, a laminin having an α4 chain and a laminin having an α5 chain, or an E8 fragment thereof, and cells adherent thereto are collected. Alternatively, the cells adherent to a laminin selected from the group consisting of a laminin having an α3 chain, a laminin having an α4 chain and a laminin having an α5 chain, or an E8 fragment thereof may not be immediately subjected to detachment or collection. For example, non-adherent cells are removed, and the adherent cells on the laminin or an E8 fragment thereof are continued to be cultured, grown and then collected. The cells collected in step (3) compose a high-purity corneal epithelial cell population that can be used for the fabrication of corneal epithelial cell sheets for transplantation.

Laminin is a heterotrimeric molecule consisting of three subunits termed α, β and γ chains. Five kinds of α chains (α1 to α5), three kinds of β chains (β1 to β3) and three kinds of γ chains (γ1 to γ3) are known, and various combinations of these chains result in at least 12 kinds of laminin isoforms (see Table 1). As used herein, each laminin isoform is written in abbreviation, for example, "laminin 111". When written simply as "laminin" herein, laminin means "full-length laminin".

[Table 1]

| α chain | Trimer composition | |
|---|---|---|
| α1 | α1β1γ1 | (laminin-1) |
| | α1β2γ1 | (laminin-3) |
| α2 | α2β1γ1 | (laminin-2) |
| | α2β2γ1 | (laminin-4) |
| | α2β1γ3 | (laminin-12) |
| α3 | α3β3γ2 | (laminin-5) |
| | α3β1γ1 | (laminin-6) |
| | α3β2γ1 | (laminin-7) |
| α4 | α4β1γ1 | (laminin-8) |
| | α4β2γ1 | (laminin-9) |
| α5 | α5β1γ1 | (laminin-10) |
| | α5β2γ1 | (laminin-11) |

The laminin E8 fragment (hereinafter referred to as "laminin E8" or "E8"), which is a heterotrimeric fragment obtained by elastase digestion of mouse laminin 111, was identified as having strong cell-adhesive activity (Edgar D et al., J. Cell Biol., 105: 589-598, 1987). Elastase digestion of laminins other than mouse laminin 111 could presumably produce fragments corresponding to the mouse laminin 111E8, but there has been no report on isolation or identification of such E8 fragments. Therefore, the laminin E8 used in the production method of the present invention does not have to be an elastase-digestion product of laminins and may be any laminin fragment equivalent in cell-adhesive activity and structure to the mouse laminin 111E8.

Laminin E8 is a trimeric fragment composed of a C-terminal fragment of the α chain lacking globular domains 4 and 5 (α chain E8), a C-terminal fragment of the β chain (β chain E8), and a C-terminal fragment of the γ chain (γ chain E8), and the molecular weight of the trimer is about 150 to 170 kDa. The molecular weight of the trimer is not particularly limited, but is usually about 150 to 170 kDa. The glutamic acid residue at the 3rd position from the C-terminus of the γ chain E8 is essential for the integrin binding activity of laminin E8 (Hiroyuki Ido et al., The Journal of Biological Chemistry, 282, 11144-11154, 2007).

The laminin may be from any organism and is preferably from mammals. The same shall apply to the laminin E8. Examples of the mammal include but are not limited to humans, mice, rats, cattle and pigs. Particularly preferred are humans. This is because, when a corneal epithelial cell population is produced for use in fabricating corneal epithelial cell sheets safe for human transplantation, the production process requires no use of xenogeneic components.

The laminin may be a native laminin or a modified laminin that has modification of one or more amino acid residues but retains biological activities of the native laminin. The same shall apply to the laminin E8. The method for producing the laminin is not particularly limited. For example, the laminin can be obtained by purification from cells highly expressing the laminin. Alternatively, the laminin can be produced as a recombinant protein. The method for producing the laminin E8 is also not particularly limited. For example, the laminin E8 can be obtained by digestion of a full-length laminin with a protease such as elastase, followed by isolation and purification of the fragment of interest. Alternatively, the laminin E8 can be produced as a recombinant protein. In terms of production quantity, quality uniformity, production cost, etc., it is preferred that the laminin and the laminin E8 are produced as recombinant proteins.

The recombinant laminin and the recombinant laminin E8 can be produced by appropriate known recombinant techniques, for example, by preparing DNAs encoding full-length laminin α, β and γ chains or DNAs encoding E8 fragments of these chains, inserting the DNAs into separate expression vectors, co-introducing the three resulting expression vectors into appropriate host cells, and purifying the expressed trimeric protein by a known method. The production method of the recombinant laminin (full-length) may be, for example, the method of Ido et al. (Hiroyuki Ido et al., The Journal of Biological Chemistry, 279, 10946-10954, 2004), but is not limited thereto. The production method of the recombinant laminin E8 may be, for example, the method of Ido et al. (Hiroyuki Ido et al., The Journal of Biological Chemistry, 282, 11144-11154, 2007), but is not limited thereto.

Information regarding the nucleotide sequences of the genes encoding laminin α, β and γ chains of major mammals and the amino acid sequences of these chains can be obtained from known databases (e.g., GenBank, etc.). The accession numbers of the constituent chains of human laminins are shown in Table 2. Information regarding the nucleotide and amino acid sequences of the constituent chains of laminins of other organisms can also be obtained from known databases (e.g., GenBank etc.).

[Table 2]

|  | Amino acid sequence | Nucleotide sequence |
|---|---|---|
| Human laminin α1 chain | NP_005550 | NM_005559 |
| Human laminin α2 chain | NP_000417 | NM_000426 |
| Human laminin α3 chain | NP_000218 | NM_000227 |
| Human laminin α4 chain | NP_002281 | NM_002290 |
| Human laminin α5 chain | NP_005551 | NM_005560 |
| Human laminin β1 chain | NP_002282 | NM_002291 |
| Human laminin β2 chain | NP_002283 | NM_002292 |
| Human laminin β3 chain | NP_000219 | NM_000228 |
| Human laminin γ1 chain | NP_002284 | NM_002293 |
| Human laminin γ2 chain | NP_005553 | NM_005562 |
| Human laminin γ3 chain | NP_006050 | NM_006059 |

Examples of the laminin having an α2 chain include laminin 211 and laminin 221. Examples of the laminin having an α3 chain include laminin 332, laminin 311 and laminin 321. Examples of the laminin having an α4 chain include laminin 411 and laminin 421. Examples of the laminin having an α5 chain include laminin 511 and laminin 521. The laminin or the laminin E8 used for collecting the non-adherent cells is preferably laminin 211E8. The laminin or the laminin E8 used for collecting the adherent cells is preferably laminin 332E8, laminin 411E8 or laminin 511E8, and more preferably laminin 332E8 or laminin 511E8.

The method for bringing the cells into contact with the laminin or the laminin E8 in step (3) is not particularly limited. For example, the culture surface of a culture vessel, such as a culture dish, is coated with the laminin or the laminin E8, and a cell suspension is added to the culture vessel and then allowed to stand for a certain period of time. In another example, a carrier, such as beads, is coated with the laminin or the laminin E8 and added to a cell suspension of the cell population containing corneal epithelial cells prepared in step (1) for contact with the cells.

The coating concentration of the laminin or the laminin E8 is not particularly limited, and any coating concentration appropriate for the purpose may be selected. For example, the coating concentration can be selected from the range of about 0.1 to 2.0 μg/cm$^2$. In the case where the laminin E8 is used for coating, the coating concentration may be about 0.25 to 1.0 μg/cm$^2$ or about 0.5 to 1.0 μg/cm$^2$. The duration time of the contact of the cells with the laminin or the laminin E8 is not particularly limited, and any duration time appropriate for the purpose in a particular coating concentration of choice may be selected. In the case where the laminin E8 is used in the production method of the present invention, the duration time is usually selected from the range of about 1 to 30 minutes. Preferred is about 3 to 20 minutes.

The method for collecting the cells not adherent to the laminin or the laminin E8 (non-adherent cells) is not particularly limited. In the case where a culture vessel coated with the laminin or the laminin E8 is used, for example, the medium is collected from the culture vessel, the coated surface is washed a few times with PBS or the like, and the collected medium and wash solution are subjected to centrifugation or the like to collect the cells contained therein. In the case where a carrier coated with the laminin or the laminin E8 has been added to a cell suspension, for example, the cell suspension after collection of the carrier is subjected to centrifugation or the like to collect the cells remaining therein.

The method for collecting cells adherent to the laminin or the laminin E8 (adherent cells) is not particularly limited, and any appropriate known method for detaching adherent cells may be selected. In the case where a culture vessel coated with the laminin or the laminin E8 is used, for example, non-adherent cells are removed, a known cell detachment solution, such as an EDTA solution or a trypsin solution, is added onto the surface coated with the laminin or the laminin E8, and adherent cells are detached by pipetting or the like and then collected. In the case where a carrier coated with the laminin or the laminin E8 is used, for example, the carrier is added to a cell detachment solution, and the adherent cells are detached by pipetting or the like and then collected. In the case where the adherent cells on a culture vessel coated with the laminin or the laminin E8 are continued to be cultured, simply removing non-adherent cells is enough.

The cell population produced by the production method of the present invention is a high-purity corneal epithelial cell population that can be directly cultured for fabrication into a corneal epithelial cell sheet for transplantation. The "corneal epithelial cell population for the fabrication of corneal epithelial cell sheets for transplantation" has to be a cell population composed of $1 \times 10^5$ cells or more, which cell number is needed for the fabrication of a corneal epithelial cell sheet for at least one eye. However, in consideration of the industrial application of the present invention, the "corneal epithelial cell population for the fabrication of corneal epithelial cell sheets for transplantation" desirably contains the number of cells needed for the fabrication of corneal epithelial cell sheets for more than one eye. Therefore, the number of cells in the "corneal epithelial cell population for the fabrication of corneal epithelial cell sheets for transplantation" is preferably $2 \times 10^5$ or more cells, $4 \times 10^5$ cells or more, $6 \times 10^5$ cells or more, $8 \times 10^5$ cells or more, or $1 \times 10^6$ cells or more. In addition, the "corneal epithelial cell population for the fabrication of corneal epithelial cell sheets for transplantation" preferably has a contaminant cell content of less than 50%, less than 45%, less than 40%, less than 35%, or less than 30%.

The corneal epithelial cell sheet for transplantation can be fabricated by seeding the cell population produced by the production method of the present invention at $1.5 \times 10^5$ to $6.0 \times 10^5$ cells/well on a 6-well plate or at $0.5 \times 10^5$ to $1.0 \times 10^5$ cells/well on a 12-well plate, followed by culturing to confluency for 5 to 12 days.

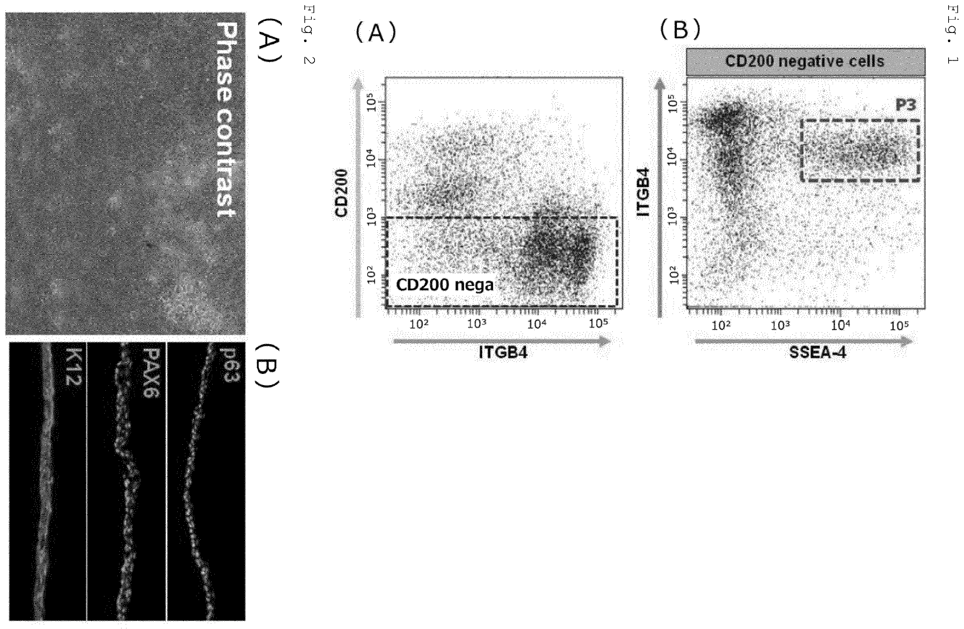

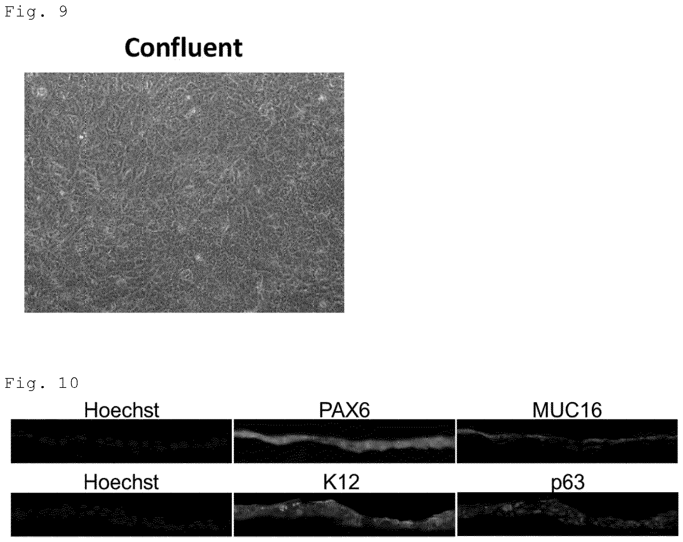

The purity of the corneal epithelial cells in the cell population produced by the production method of the present invention can be determined by measuring the number of the CD200-negative/SSEA-4-positive/ITGB4-positive cells relative to the total cell number of the cell population. However, in the case where the cells adherent to the laminin or the laminin E8 in step (3) of the production method of the present invention are detached from dishes and subjected to FACS sorting, the marker proteins on the cell surface may be damaged by cell detachment enzymes etc. and thus fail to be recognized by their respective specific antibodies. In this case, the number of the CD200-negative/SSEA-4-positive/ITGB4-positive cells cannot be exactly measured. For this reason, in the present invention, the purity of the corneal epithelial cells may be determined based on the proportion of PAX6-positive/p63-positive cells in the cells adherent to the laminin or the laminin E8 in step (3). PAX6 is an eye-related transcription factor, and p63 is known as an epithelial stem cell marker. The present inventors have confirmed that a cell sheet fabricated from CD200-negative/SSEA-4-positive/ITGB4-positive cells is positive for both PAX6 and p63 (see Fig. 2 of Reference Example 1 and Fig. 10 of Example 1).

The method for determining the proportion of PAX6-positive/p63-positive cells in the cells adherent to the laminin or the laminin E8 is not particularly limited. For example, the adherent cells are fixed with paraformaldehyde or the like and stained with an anti-PAX6 antibody and an anti-p63 antibody, and the nuclei are stained with Hoechst 33342, DAPI or the like. The total number of the nuclear stained cells and the number of the cells positive for both PAX6 and p63 are counted to calculate the proportion of PAX6-positive/p63-positive cells. The proportion of PAX6-positive/p63-positive cells in the cells adherent to the laminin or the laminin E8 is required to be 50% or more and may be 55% or more, 60% or more, 65% or more, or 70% or more.

The production method of the present invention enables less time-consuming mass production of a corneal epithelial cell population having a purity comparable to that of the corneal epithelial cell population prepared by separation of CD200-negative/SSEA-4-positive/ITGB4-positive cells by FACS sorting. For example, in the case where a corneal epithelial cell population for the fabrication of a corneal epithelial cell sheet for transplantation is produced by subjecting a cell population having a corneal epithelial cell content of about 5% to FACS sorting using SONY Cell Sorter SH800, $1 \times 10^5$ CD200-negative/SSEA-4-positive/ITGB4-positive cells are required for the fabrication of a corneal epithelial cell sheet for one eye. To this end, the cell population before FACS sorting is required to contain $2 \times 10^6$ cells or more, and the sorting takes about 30 minutes to about 1 hour. In contrast, in the case of the production method of the present invention, namely, the method using a combined technique of MACS and cell adhesion to the laminin or the laminin E8, the cell number in a cell population processable by a commercial magnetic-activated cell sorter is 1 or 2 orders of magnitude greater than that processible by FACS ($2 \times 10^6$ cells), and moreover, the process time is shorter. The step of cell adhesion to the laminin or the laminin E8 can also be quickly performed on a large amount of cells by adequately increasing the number of plates. Therefore, the production method of the present invention is a very useful production method which is more suitable for practical and industrial use than the FACS sorting-based method.

Moreover, the production method of the present invention enables production of a safer and less damaged corneal epithelial cell population than that obtained by FACS sorting-based purification. Cells collected after FACS sorting are known to be heavily damaged, and there has been a desire for a method which, without using FACS sorting, can produce a cell population having a purity comparable to that achievable by FACS sorting. The production method of the present invention is characterized by a combined technique of MACS and cell adhesion to the laminin or the laminin E8, which technique allows production of a corneal epithelial cell population having a purity comparable to that achievable by FACS sorting. In addition, the production method of the present invention, which comprises the step of collecting cells adherent to a specific laminin or laminin E8, is expected to allow activation and increased viability of the cells. The cells adhere to the laminin or the laminin E8 via a specific integrin on the cell surface. Such integrin-dependent cell-laminin adhesion is known to prevent cell apoptosis and maintain cell survival as well as activate intracellular signaling pathways for cell growth promotion (Gu J et al. The Journal of Biological Chemistry 277:19922-19928, 2002). Therefore, the production method of the present invention is greatly advantageous over the FACS sorting-based method in that the resulting corneal epithelial cell population has a high viability.

The corneal epithelial cell population produced by the production method of the present invention can be directly used as a material of corneal epithelial cell sheets for transplantation. Therefore, the present invention includes a method for fabricating a corneal epithelial cell sheet for transplantation, which method comprises the step of culturing the corneal epithelial cell population produced by the production method of the present invention. There is no particular limitation on the culture method for the fabrication of a corneal epithelial cell sheet for transplantation from the corneal epithelial cell population produced by the production method of the present invention. For example, the cell population produced by the production method of the present invention is seeded at $1.5 \times 10^5$ to $6.0 \times 10^5$ cells/well on a 6-well plate or at $0.5 \times 10^5$ to $1.0 \times 10^5$ cells/well on a 12-well plate, and cultured to confluency for 5 to 12 days. Medium replacement is preferably performed once every two days. The plates may be typical cell culture plates. Alternatively, a temperature-responsive cell cultureware for cell sheet engineering ("UpCell (trade name)" manufactured by CellSeed) may be used.

EXAMPLES

Hereinafter, the present invention will be described in detail by examples, but the present invention is not limited thereto.

Reference Example 1:

Separation of CD200-negative/SSEA-4-positive/ITGB4-positive cells by FACS sorting and analysis of corneal epithelial cell markers

(1) Induced differentiation of iPS cells into periocular cells

For induced differentiation of iPS cells into periocular cells, the protocol for self-formed ectodermal autonomous multi-zone (SEAM) formation (Hayashi et al. Nature. 2016 Mar 17; 531 (7594):376-80.) was used. Human iPS cells (201B7) were seeded at a density of 300 to 440 cells/cm$^2$ on 6- or 12-well plates coated with laminin 511E8 (i-Matrix; Nippi) at a concentration of 0.5 $\mu$g/cm$^2$, maintained for 8 to 10 days in StemFit medium (Ajinomoto), and cultured for 4 weeks in a differentiation medium (DM; GMEM (Life Technologies) supplemented with 10% knockout serum replacement (KSR, Life technologies), 1 mM sodium pyruvate (Life Technologies), 0.1 mM non-essential amino acids (Life Technologies), 2 mM L-glutamine (Life Technologies), 1% penicillin-streptomycin solution (Life Technologies) and 55 $\mu$M 2-mercaptoethanol (Life Technologies)). After that, culture was continued for 4 weeks in corneal differentiation medium (CDM; a 1:1 mixed medium of DM supplemented with 20 ng/mL KGF (Wako), 10 $\mu$M Y-27632 (Wako) and 1% penicillin-streptomycin solution and Cnt-20 or Cnt-PR (without EGF or FGF2) (CeLLnTEC Advanced Cell Systems)) and subsequently for additional 2 to 5 weeks in corneal epithelial cell maintenance medium (CEM; DMEM/F12 (2:1) (Life Technologies) supplemented with 2% B27 supplement (Life Technologies), 1% penicillin-streptomycin, 20 ng/mL KGF and 10 $\mu$M Y-27632).

(2) Preparation of a suspension containing periocular cells derived by induced differentiation

After induced differentiation, the cells were treated with Accutase (Life Technologies) and resuspended in KCM medium (DMEM/F-12 supplemented with 5% FBS (Japan Bio Serum, Hiroshima, Japan), 0.4 $\mu$g/mL hydrocortisone

succinate (Wako), 2 nM 3,3',5-triiodo-L-thyronine sodium salt (MP biomedicals, Santa Ana, CA), 1 nM cholera toxin (List Biological Laboratory, Campbell, CA), 2.25 μg/mL bovine transferrin HOLO form (Life Technologies), 2 mM L-glutamine, 0.5% insulin transferrin selenium solution (Life Technologies) and 1% penicillin-streptomycin, without glutamine (Thermo Fisher Scientific)). The cell suspension was filtered through a cell strainer (40 μm, BD Biosciences, San Diego, CA), and a periocular cell suspension was prepared in PBS and subjected to the subsequent steps.

(3) Separation of CD200-negative/SSEA-4-positive/ITGB4-positive cells by FACS sorting

The periocular cell suspension prepared in the above (2) was stained with a PE-Cy7-labeled anti-CD200 antibody (BD Pharmingen), an FITC-labeled anti-SSEA-4 antibody (BioLegend) and an Alexa Fluor 647-labeled anti-CD104 antibody (anti-ITGB4 antibody) (BioLegend or BD Pharmingen). The stained cells were processed on FACS Verse (BD Biosciences) to collect CD200-negative cells (area enclosed by the dashed line in Fig. 1A) and further collect SSEA-4- and ITGB4-positive cells (P3 area enclosed by the dashed line in Fig. 1B) as shown in Fig. 1.

(4) Morphology of CD200-negative/SSEA-4-positive/ITGB4-positive cells and expression of corneal epithelial markers

The collected CD200-negative/SSEA-4-positive/ITGB4-positive cells were seeded at $1 \times 10^5$ cells/well on 12-well plates coated with laminin 511E8 (i-Matrix; Nippi) at a concentration of 0.5 μg/cm$^2$ and cultured in the corneal epithelial cell maintenance medium for 3 weeks. After the morphology of the cells was observed with a phase-contrast microscope, the medium was removed, and the cells were washed with PBS and incubated in DMEM containing 2.4 U/mL dispase (Life Technologies) at 37°C for 10 minutes. The medium was removed, the cell sheet was collected using a PVDF membrane ring and embedded in OCT compound, and cryosections were prepared. The sections were mounted on glass slides and incubated with primary antibodies (an anti-K12 antibody (N-16, Santa Cruz), an anti-PAX6 antibody (PRB-278P, Covance) and an anti-p63 antibody (4A4, Santa Cruz)) at 4°C overnight. After TBS washing, the sections on the glass slides were incubated with secondary antibodies labeled with Alexa Fluor 488, 568 and 647 at room temperature for 1 hour. After TBS washing, the sections were observed with a fluorescence microscope (Axio Observer D1).

(5) Results

The results are shown in Fig. 2. Fig. 2A shows the results of the phase-contrast microscopic observation of the cells cultured for 3 weeks. Fig. 2B shows the results of the analysis of marker gene expression in the corneal epithelial cell sheet. As shown in Fig. 2A, the phase-contrast microscopic observation after 3-week culture revealed that homogeneous cells were stratified to form a sheet. No contaminant cell colonies were observed. In addition, as shown in Fig. 2B, the resulting cell sheet expressed the epithelial stem cell marker p63, the eye-related transcription factor PAX6, and the corneal epithelium-specific marker K12 (keratin 12), demonstrating the formation of a corneal epithelial cell sheet.

Reference Example 2:

Purification of corneal epithelial cells by MACS

(1) Antibody staining

The periocular cell suspension prepared in Reference Example 1 (1) and (2) was stained with a PE-Cy7-labeled anti-CD200 antibody (BD Pharmingen), an FITC-labeled anti-SSEA-4 antibody (BioLegend) and a PE-labeled anti-CD104 antibody (anti-ITGB4 antibody) (BioLegend or BD Pharmingen). The stained cells were resuspended in MACS buffer (PBS with 0.5% BSA and 2 mM EDTA) and processed on a magnetic-activated cell sorter (Miltenyi Biotec). For depletion of CD200-positive cells, the stained cells were labeled with anti-Cy7 MicroBeads (Miltenyi Biotec) at 4°C for 15 minutes, washed with MACS buffer, and applied to an MS or LS column (Miltenyi Biotec) in a magnetic field for removal of labeled cells and collection of unlabeled cells. For isolation of SSEA-4-positive cells, the collected unlabeled cells were labeled with anti-FITC MicroBeads (Miltenyi Biotec) at 4°C for 15 minutes, washed with MACS buffer, and applied to an MS or LS column in a magnetic field. Subsequently, the column was rinsed with MACS buffer and departed from the magnetic field, and the cells trapped on the column were collected in MACS buffer.

(2) FACS analysis

The antibody-stained cell population before MACS, the cell population depleted of CD200-positive cells by MACS, and the SSEA-4-positive cell population separated by MACS from the CD200-positive cell-depleted cell population were analyzed with Cell Sorter SH800 (SONY) or FACS Verse (BD Biosciences), and the percentage of cells positive for each marker was determined.

(3) Results

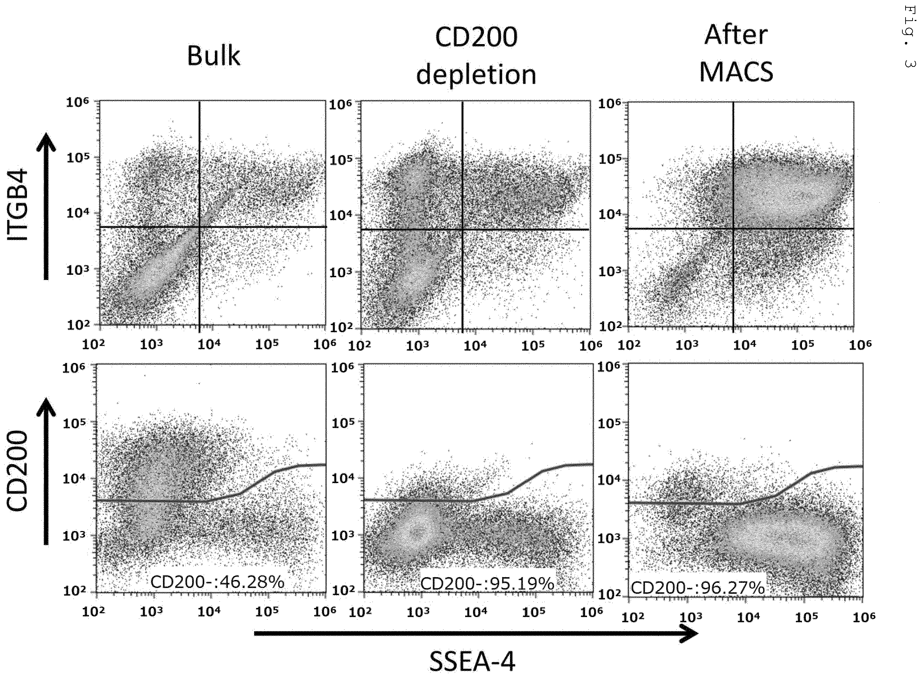

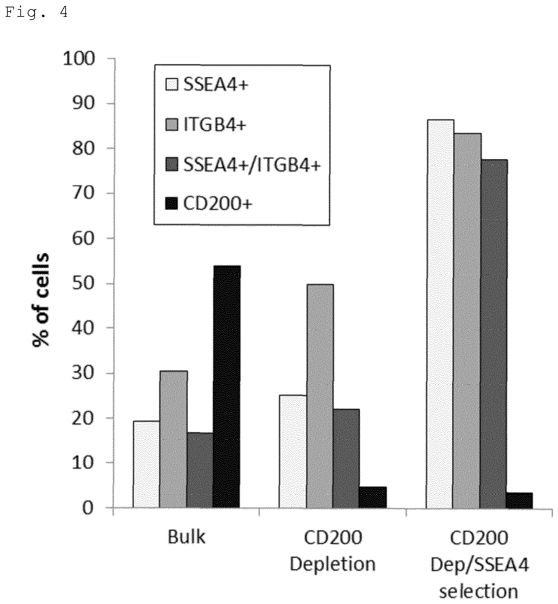

The results are shown in Figs. 3 and 4. Figs. 3 and 4 show the results of FACS analysis. In the figures, "Bulk" represents the results for the antibody-stained cell population before MACS, "CD200 Depletion" represents the results for the cell population depleted of CD200-positive cells by MACS, and "After MACS" (Fig. 3) and "CD200 Dep/SSEA4 selection" (Fig. 4) each represent the results for the SSEA-4-positive cell population separated by MACS from the CD200-positive cell-depleted cell population. In Fig. 4, "SSEA4+" represents the percentage of the cells present on the right side of the vertical line of each histogram at the top row of Fig. 3; "ITGB4+" represents the percentage of the cells present above the transversal line of each histogram at the top row of Fig. 3; "SSEA4+/ITGB4+" represents the percentage of the cells present in the upper right section of each histogram at the top row of Fig. 3; and "CD200+" represents the percentage of the cells present above the transversal curve of each histogram at the bottom row of Fig. 3. As is clear from Figs. 3 and 4, in the SSEA-4-positive cell population separated by MACS from the CD200-positive cell-depleted cell population, SSEA-4/ITGB4-positive cells were present in a higher percentage, but CD200-positive cells still remained.

Reference Example 3:

Purification of corneal epithelial cells with use of laminin E8s

(1) Laminin E8s

Laminin 111E8 (hereinafter referred to as "LN111E8", and other laminin E8s are similarly abbreviated), LN211E8, LN332E8, LN411E8 and LN511E8, that is, 5 kinds of laminin E8s were used. The LN511E8 was a commercial product (i-Matrix; Nippi), and other laminin E8s were received from Professor Kiyotoshi Sekiguchi from Osaka University.

(2) Plate coating

To 12-well plates (BD Falcon), 1 mL/well of PBS was added, and the E8 fragment of each laminin isoform was added such that the coating concentration would be 0.5 $\mu$g/cm$^2$. The plates were allowed to stand at 37°C for 1 to 3 hours.

(3) FACS analysis of non-adherent cells

As described in Reference Example 2 (1), the periocular cell suspension prepared in Reference Example 1 (1) and (2) was stained with a PE-Cy7-labeled anti-CD200 antibody (BD Pharmingen), an FITC-labeled anti-SSEA-4 antibody (BioLegend) and a PE-labeled anti-CD104 antibody (anti-ITGB4 antibody) (BioLegend or BD Pharmingen) . The stained cells were suspended in the corneal epithelial cell maintenance medium and seeded at $5 \times 10^5$ cells/well on the coated plates described above. For the control, the stained cells were seeded on a non-coated plate. After incubation at 37°C for 5 to 30 minutes, non-adherent cells were collected. The collected cells were resuspended in PBS and subjected to FACS analysis with Cell Sorter SH800 (SONY). The present inventors previously confirmed that the binding of the cells to the anti-ITGB4 antibody used in this experiment does not influence cell-laminin E8 adhesion.

(4) Results

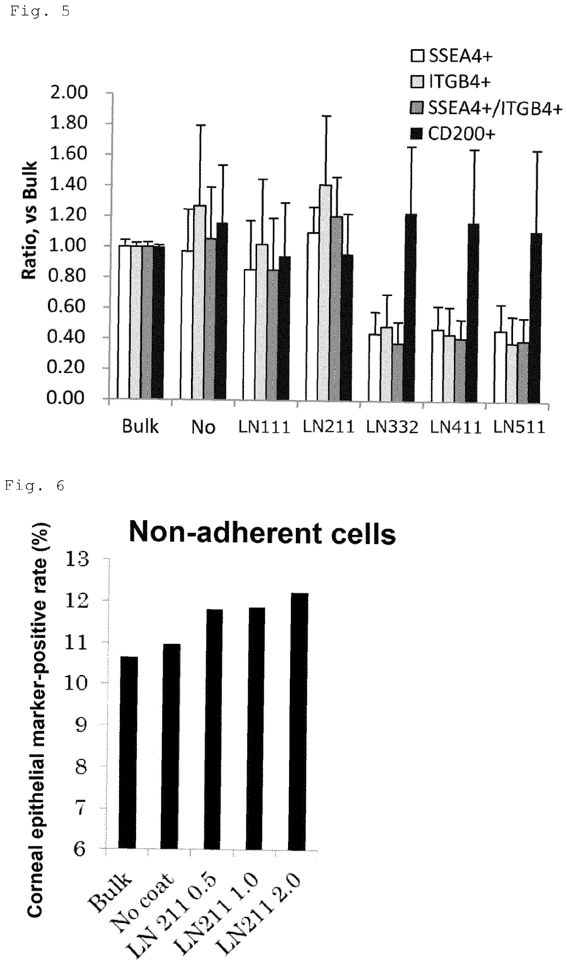

The results of the FACS analysis of non-adherent cells are shown in Fig. 5. In the figure, "Bulk" stands for an antibody-stained cell population not plated, and "No" stands for a cell population seeded on the non-coated plate. The results are represented as the ratio of the percentage of the SSEA4+, ITGB4+, SSEA4+/ITGB4+ or CD200+ fraction in each group relative to the corresponding percentage in the Bulk group. As is clear from Fig. 5, in the cells not adherent to LN332E8 (LN332 in the figure), LN411E8 (LN411 in the figure) or LN511E8 (LN511 in the figure), the percentage of CD200-positive cells was higher than that in the Bulk group, and the percentages of SSEA4-positive cells, ITGB4-positive cells and SSEA4/ITGB4-positive cells were lower than the corresponding percentages in the Bulk group. In contrast, in the cells not adherent to LN211E8 (LN211 in the figure), the percentage of CD200-positive cells was equivalent to that in the Bulk group, and the percentages of ITGB4-positive cells and SSEA4/ITGB4-positive cells tended to be higher than the corresponding percentages in the Bulk group. The composition pattern of the non-adherent cells in the No group and the composition pattern of the cells not adherent to LN111E8 (LN111 in the figure) were almost equivalent to that in the Bulk group. These results indicate that the depletion of cells not adherent to LN332E8, LN411E8 or LN511E8 can enhance the purity of SSEA4/ITGB4-positive cells.

Reference Example 4:

Purification of corneal epithelial cells with use of LN211E8

(1) Plate Coating

To 12-well plates (BD Falcon), 1 mL/well of PBS was added, and LN211E8 was added such that the coating concentrations would be from 0.5 $\mu$g/cm$^2$ to 5.0 $\mu$g/cm$^2$. The plates were allowed to stand at 37°C for 1 to 3 hours.

(2) Percentage of corneal epithelial cells in non-adherent cells

As described in Reference Example 2 (1), the periocular cell suspension prepared in Reference Example 1 (1) and (2) was stained with a PE-Cy7-labeled anti-CD200 antibody (BD Pharmingen), an FITC-labeled anti-SSEA-4 antibody (BioLegend) and a PE-labeled anti-CD104 antibody (anti-ITGB4 antibody) (BioLegend or BD Pharmingen) . The stained cells were suspended in the corneal epithelial cell maintenance medium and seeded at $5 \times 10^5$ cells/well on the coated plates described above. For the control, the stained cells were seeded on a non-coated plate. After incubation at 37°C for 10 minutes, non-adherent cells were collected. The collected cells were resuspended in PBS and subjected to FACS analysis with Cell Sorter SH800 (SONY) to determine the percentage of CD200-negative/SSEA-4-positive/ITGB4-positive cells (the corneal epithelial marker-positive rate).

(3) Results

The results are shown in Fig. 6. In the figure, "Bulk" stands for an antibody-stained cell population not plated. The corneal epithelial marker-positive rate in the cells not adherent to LN211E8 was higher than that in the cell population before purification and higher than that of the cells collected from the non-coated plate. These results indicate that the collection of cells not adherent to LN211E8 can enhance the purity of SSEA4/ITGB4-positive cells.

(4) Gene expression analysis of adherent cells

After the collection of the non-adherent cells, RNA was extracted with QIAzol Lysis Reagent (QIAGEN) from the remaining adherent cells on each plate. cDNA was synthesized from the extracted RNA with SuperScript III First-Strand Synthesis SuperMix for qRT-PCR (Invitrogen) in the 20-$\mu$L reaction system and subjected to qRT-PCR using TaqMan Assays (Life Technologies) . The expression of K12 and TP63 as marker genes for corneal epithelial cells was analyzed. The expression of ITGA7 and vimentin as marker genes for non-corneal epithelial cells was analyzed.

(5) Results

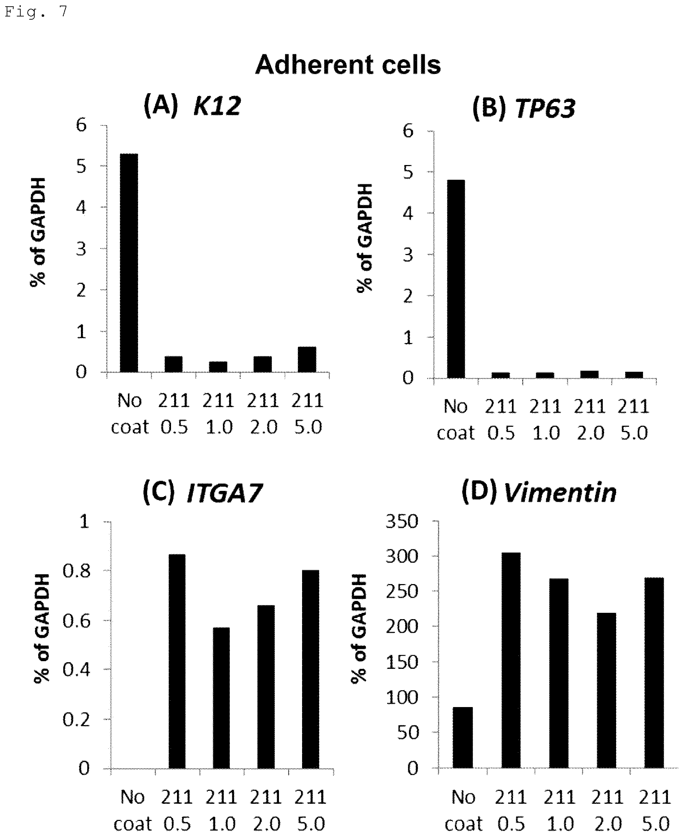

The results are shown in Fig. 7. Fig. 7A shows the results for K12, Fig. 7B shows the results for TP63, Fig. 7C shows the results for ITGA7, and Fig. 7D shows the results for vimentin. In the cells adherent to LN211E8 (211 in the figure), the expression levels of K12 and TP63 as marker genes for corneal epithelial cells were low, and the expression levels of ITGA7 and vimentin as marker genes for non-corneal epithelial cells were high. These results show that the depletion of cells adherent to LN211E8 and the collection of cells not adherent to LN211E8 using LN211E8-coated plates can enhance the purity of SSEA4/ITGB4-positive cells.

Example 1:

Purification of corneal epithelial cells by a combined technique of MACS and cell-laminin E8 adhesion

1-1 Purification of corneal epithelial cells

(1) Purification by MACS

The periocular cell suspension prepared in Reference Example 1 (1) and (2) was stained with a PE-Cy7-labeled anti-CD200 antibody (BD Pharmingen). The stained cells were resuspended in MACS buffer and processed on a magnetic-activated cell sorter (Miltenyi Biotec). First, the stained cells were depleted of CD200-positive cells, and SSEA-4-positive cells were then isolated. For depletion of CD200-positive cells, the stained cells were labeled with anti-Cy7 MicroBeads (Miltenyi Biotec) at 4°C for 15 minutes, washed with MACS buffer, and applied to an MS or LS column (Miltenyi Biotec) in a magnetic field for removal of labeled cells and collection of unlabeled cells. For isolation of SSEA-4-positive cells, the collected unlabeled cells were labeled with anti-SSEA4 MicroBeads (Miltenyi Biotec) at 4°C for 15 minutes, washed with MACS buffer, and applied to an MS or LS column in a magnetic field. Subsequently, the column was rinsed with MACS buffer and departed from the magnetic field, and the cells trapped on the column were collected in MACS buffer.

(2) Purification by cell-laminin E8 adhesion

The cells after purification by MACS were seeded at about $5 \times 10^5$ cells/well on an LN511E8-coated plate (coating concentration: 0.5 $\mu$g/cm$^2$), incubated at 37°C for 5 minutes, and depleted of non-adherent cells.

1-2 Gene expression analysis

(1) Experimental method

The expression of marker genes for corneal epithelial cells and marker genes for non-corneal epithelial cells in the cell population at different stages was analyzed. More specifically, the expression analysis was performed on the periocular cell population prepared in Reference Example 1 (1) and (2) (Bulk), the cell population depleted of CD200-positive cells by MACS (CD200 Dep.), the SSEA-4-positive cell population isolated by MACS from the CD200-positive cell-depleted cell population (After MACS1), and the population of cells adherent to the LN511E8-coated plate after MACS (MLS (511)). As the control, a CD200-negative/SSEA-4-positive/ITGB4-positive-cell population purified by FACS was used. RNA was extracted with QIAzol Lysis Reagent (QIAGEN) from each cell population. cDNA was synthesized from the extracted RNA with SuperScript III First-Strand Synthesis SuperMix for qRT-PCR (Invitrogen) in the 20-$\mu$L reaction system and subjected to qRT-PCR using TaqMan Assays (Life Technologies). The expression of K12, PAX6, TP63 and ITGB4 as marker genes for corneal epithelial cells was analyzed. The expression of CRYAA, RPE65, CD200 and K10 as marker genes for non-corneal epithelial cells was analyzed.

(2) Results

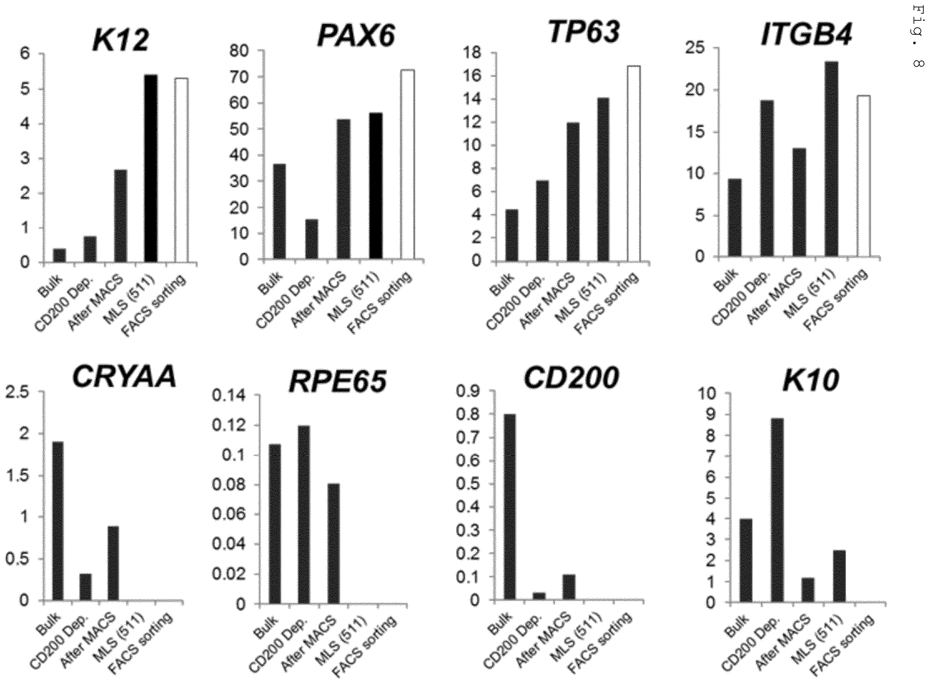

The results are shown in Fig. 8. The top row (K12, PAX6, TP63, ITGB4) shows the results for the marker genes for corneal epithelial cells, and the bottom row (CRYAA, RPE65, CD200, K10) shows the results for the marker genes for non-corneal epithelial cells. In the cell population purified by the combined technique of MACS and cell-laminin E8 adhesion (MLS (511) in the figure), the expression levels of the marker genes for corneal epithelial cells (top row) were almost comparable to those in the control, and the expression levels of the marker genes for non-corneal epithelial cells (bottom row) were below the detection limit, except that K10 was slightly expressed.

1-3 Fabrication of corneal epithelial cell sheet

(1) Experimental method

The cells adherent to the LN511E8-coated plate in the above (2) were continued to be cultured for 3 weeks . The culture grown to confluency was composed of homogeneous cells. The results of the phase-contrast microscopic observation of cell morphology are shown in Fig. 9.
The medium was removed, and the cells were washed with PBS and incubated in DMEM containing 2.4 U/mL dispase (Life Technologies) at 37°C for 10 minutes. The medium was removed, the cell sheet was collected using a PVDF membrane ring and embedded in OCT compound, and cryosections were prepared. The sections were mounted on glass slides and incubated with primary antibodies (an anti-K12 antibody (N-16, Santa Cruz), an anti-PAX6 antibody (PRB-278P, Covance), an anti-MUC16 antibody (OV185:1, abcam) and an anti-p63 antibody (4A4, Santa Cruz)) at 4°C overnight. After TBS washing, the sections on the glass slides were incubated with secondary antibodies labeled with Alexa Fluor 488, 568 and 647 at room temperature for 1 hour. Counterstaining with Hoechst 33342 (Molecular Probes) was performed at room temperature for 10 minutes . After TBS washing, the sections were observed with a fluorescence microscope (Axio Observer D1).

(2) Results

The results are shown in Fig. 10. The resulting cell sheet was positive for all four markers, namely, K12, PAX6, MUC16 and p63, demonstrating the formation of a corneal epithelial cell sheet.

1-4 FACS Analysis of cell composition of corneal epithelial cell sheets

(1) Experimental method

The cells cultured for 3 weeks on the LN511E8-coated plate in the above 1-3 were incubated with Accutase at 37°C for 1 hour, detached, and dissociated into single cells. After PBS washing, the cells were fixed by incubation with BD Cytofix/Cytoperm solution (BD Biosciences) at 4°C for 20 minutes. The cells were washed with and resuspended in BD Perm/wash buffer (BD Biosciences), and stained with primary antibodies (an anti-K12 antibody (N-16, Santa Cruz) and an anti-K14 antibody (RCK107, abcam)) and the corresponding fluorescent-labeled secondary antibodies . The cells were washed with and resuspended in PBS, and analyzed with FACS Verse (BD Biosciences). In the case where the primary antibody was the anti-K12 antibody, the cells were reacted with the primary antibody at 4°C for 3 hours and then reacted with the secondary antibody at 4°C for 1 hour. In the case where the primary antibody was the anti-K14 antibody, the primary and secondary antibodies were concurrently added and allowed to react with the cells at room temperature for 2 hours.

For the control, CD200-negative/SSEA-4-positive/ITGB4-positive cells that were separated by FACS sorting and cultured on an LN511E8-coated plate for 3 weeks (see Reference Example 1) were subjected to detachment, dissociation, fixation, antibody staining and analysis with FACS Verse (BD Biosciences) in the same manner as described above.

(2) Results

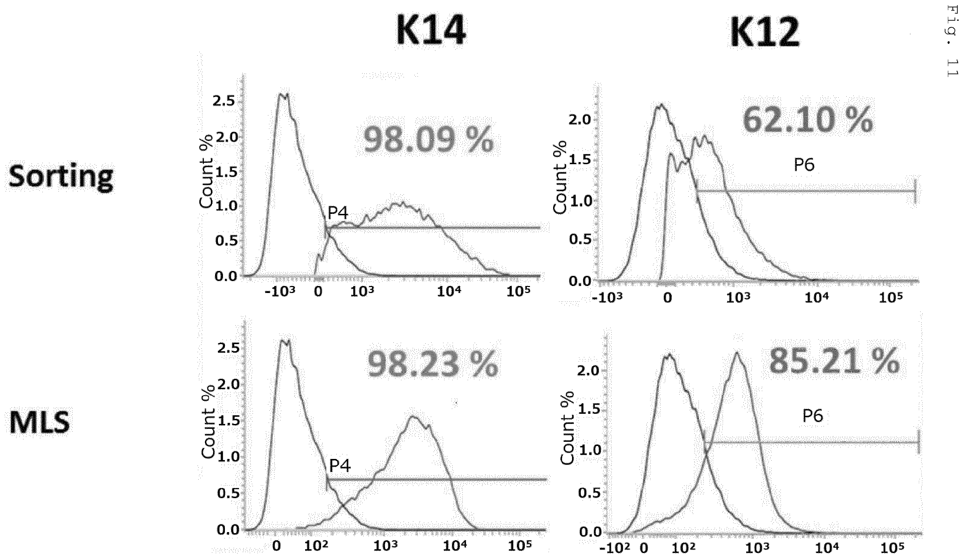

The results are shown in Fig. 11. The top row (Sorting) shows the results for the CD200-negative/SSEA-4-positive/ITGB4-positive cells separated by FACS sorting, and the bottom row (MLS) shows the results for the cell population obtained by the method of the present invention. The peak on the left in each histogram is a peak of the isotype control. The K14 (stratified epithelium marker) and K12 (corneal epithelium-specific keratin) positive rates in the cell sheet fabricated from the cell population obtained by the method of the present invention were equivalent to or higher than those in the cell sheet fabricated from the cell population obtained by FACS sorting.

1-5 Phase-contrast microscopic observation of corneal epithelial cell sheets

(1) Experimental method

The six kinds of cells shown below were cultured for 3 weeks and fabricated into cell sheets, and the cell sheets were observed with a phase-contrast microscope.

(A) MACS Only

Cells collected as described in the above 1-1 (1) were seeded at $1 \times 10^5$ cells/well on a 12-well plate coated with LN511E8 (i-Matrix; Nippi) at a concentration of 0.5 $\mu$g/cm$^2$ and cultured in the corneal epithelial cell maintenance medium for 3 weeks.

(B) LN332 Only

The periocular cell suspension prepared in Reference Example 1 (1) and (2) was seeded at about $5 \times 10^5$ cells/well on a 12-well plate coated with LN332E8 at a concentration of 0.5 $\mu$g/cm$^2$ and incubated at 37°C for 15 minutes. After removal of non-adherent cells, adherent cells were continued to be cultured in the corneal epithelial cell maintenance medium for 3 weeks.

(C) LN511 Only

The periocular cell suspension prepared in Reference Example 1 (1) and (2) was seeded at about $5 \times 10^5$ cells/well on a 12-well plate coated with LN511E8 at a concentration of 0.5 $\mu$g/cm$^2$ and incubated at 37°C for 15 minutes. After removal of non-adherent cells, adherent cells were continued to be cultured in the corneal epithelial cell maintenance medium for 3 weeks.

(D) Sorting

CD200-negative/SSEA-4-positive/ITGB4-positive cells separated by FACS sorting were seeded at $1 \times 10^5$

cells/well on a 12-well plate coated with LN511E8 at a concentration of 0.5 μg/cm² and cultured in the corneal epithelial cell maintenance medium for 3 weeks (see Reference Example 1).

(E) MLS(322): the corneal epithelial cell population obtained by the production method of the present invention

Cells collected as described in the above 1-1 (1) were seeded at about 5 × 10⁵ cells/well on a 12-well plate coated with LN332E8 at a concentration of 0.5 μg/cm² and incubated at 37°C for 5 minutes. After removal of non-adherent cells, adherent cells were continued to be cultured in the corneal epithelial cell maintenance medium for 3 weeks.

(F) MLS (511) : the corneal epithelial cell population obtained by the production method of the present invention

Cells collected as described in the above 1-1 (1) were seeded at about 5 × 10⁵ cells/well on a 12-well plate coated with LN511E8 at a concentration of 0.5 μg/cm² and incubated at 37°C for 15 minutes. After removal of non-adherent cells, adherent cells were continued to be cultured in the corneal epithelial cell maintenance medium for 3 weeks.

(2) Results

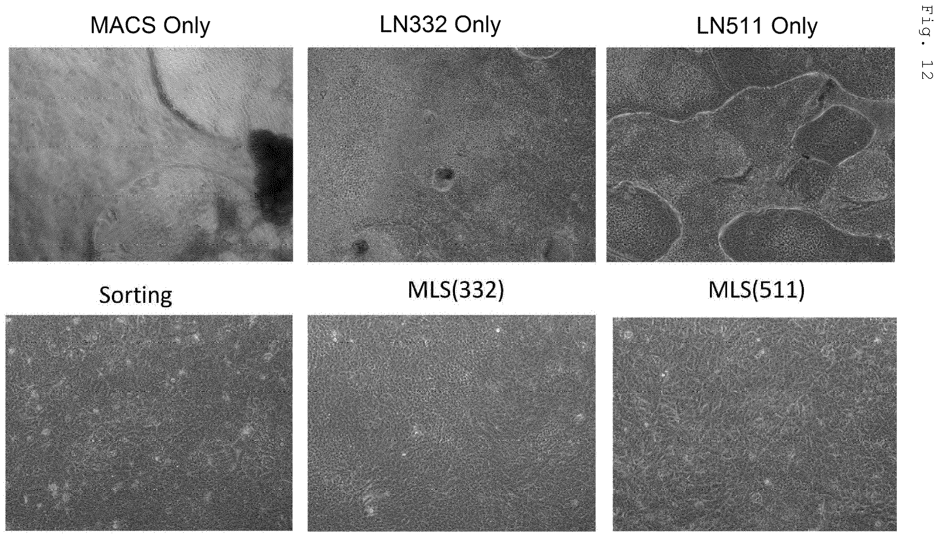

The results are shown in Fig. 12. In the top panels (A) MACS Only, (B) LN332 Only and (C) LN511 Only, contaminant cell colonies were observed in the field of view, but in the panels (D) Sorting, (E) MLS(322) and (F) MLS(511), no contaminant cell colonies were observed in the cell sheet.

1-6 Evaluation of purification efficiency

(1) Calculation method of purification efficiency

The percentages (%) of contaminant cells, i.e., cells other than the CD200-negative/SSEA-4-positive/ITGB4-positive cells (corneal epithelial cells) in the cell populations before and after MACS in Reference Example 2 were determined by FACS. From the percentages of contaminant cells in the cell populations before and after MACS, purification efficiency (contaminant-cell residual rate) was calculated according to the following formula.

```
Purification efficiency = percentage (%) of contaminant cells

after MACS/percentage (%) of contaminant cells before MACS ×

100
```

The percentages (%) of contaminant cells, i.e., cells other than the CD200-negative/SSEA-4-positive/ITGB4-positive cells (corneal epithelial cells) in the cell populations before and after laminin E8-mediated purification (cell-laminin E8 adhesion-based purification) in Reference Example 3 were determined by FACS. More specifically, the cell population before purification was seeded at 5 × 10⁵ cells/well on a 12-well plate (BD Falcon) coated with LN332E8, LN411E8 or LN511E8 at a concentration of 0.5 μg/cm², incubated at 37°C for 5 minutes, and depleted of non-adherent cells. Adherent cells were continued to be cultured for 3 weeks, detached, dissociated into single cells, and subjected to antibody staining and FACS analysis. From the percentages of contaminant cells in the cell populations before and after purification, purification efficiency (contaminant-cell residual rate) was calculated according to the following formula.

```
Purification efficiency = percentage (%) of contaminant cells

in the cell population subjected to cell-laminin E8

adhesion-based purification and subsequent 3-week

culture/percentage (%) of contaminant cells before

purification × 100
```

The theoretical value of the purification efficiency of the cell population subjected to MACS and subsequent cell-laminin E8 adhesion followed by 3-week culture was determined by multiplication of the above respective calculated purification efficiencies. The actual value was determined by counting contaminant cell colonies visually observed in the cell sheet after 3-week culture.

(2) Results

The results are shown in Table 3. MACS reduced the contaminant cell content to 26.75% of the level before the separation. Meanwhile, cell-laminin E8 adhesion, only when the most effective LN332E8 was used, reduced the contaminant cell content to 74.14% of the level before the separation, but when LN411E8 or LN511E8 was used, no reduction in the contaminant cell content was shown. The theoretical value of the purification efficiency calculated from these found values was 19.83% for the combination of MACS and LN332E8 and 27.37% for the combination of MACS and LN511E8, but visual observation showed no contaminant cell colony (contaminant-cell residual rate: 0%) in the cell sheet formed after 3-week culture in either case.

These results demonstrate that, in the case where cell-laminin E8 adhesion-based purification is applied to a periocular cell suspension which is prepared by induced differentiation and contains a large number of contaminant cells, i.e., cells other than corneal epithelial cells (see Reference Example 1), purification efficiency is low, but in the case where cell-laminin E8 adhesion-based purification is applied to a cell population in which the contaminant cell content has been reduced to about 20% by MACS, almost complete depletion of contaminant cells can be achieved.

[Table 3]

| Purification method | | Percentage of contaminant cells (%) | | Purification efficiency (%) (contaminant-cell residual rate) |
|---|---|---|---|---|
| | | Bulk | After purification | |
| MACS-based purification | | 83.39 | 22.31 | 26.75 |
| Laminin E8 adhesion-based purification + 3-week culture | LN332E8 | 80.95 | 60.02 | 74.14 |
| | LN411E8 | 80.95 | 82.24 | 101.59 |
| | LN511E8 | 80.95 | 82.81 | 102.30 |
| MACS + LN332E8 adhesion + 3-week culture (theoretical value) | | 26.75 × 74.14 | | 19.83 |
| MACS + LN332E8 adhesion + 3-week culture (actual value) | | | | 0 (no contaminant cell colony) |
| MACS + LN511E8 adhesion + 3-week culture (theoretical value) | | 26.75 × 102.30 | | 27.37 |
| MACS + LN511E8 adhesion + 3-week culture (actual value) | | | | 0 (no contaminant cell colony) |

Example 2:

Quantification of p63-positive cells in a corneal epithelial cell population purified by a combined technique of MACS and cell-laminin E8 adhesion

(1) Experimental method

Periocular cells were prepared by induced differentiation as described in Reference Example (1) from epithelial stem cell marker p63 knock-in iPS cells in which p63 and EGFP are co-expressed (Stem Cell Res. 2017 Oct 31;25:256-265. doi: 10.1016/j.scr.2017.10.015.), and a periocular cell suspension was prepared as described in Reference Example (2).

The periocular cells were seeded at $1 \times 10^5$ cells/well on a 12-well plate (BD Falcon) coated with LN211E8 at a concentration of 1 $\mu$g/cm$^2$ and incubated at 37°C for 15 minutes. Non-adherent cells were collected and seeded on a 12-well plate (BD Falcon) coated with LN332E8 or LN511E8 at a concentration of 1 $\mu$g/cm$^2$. More specifically, non-adherent cells collected from individual wells of the LN211E8-coated plate were seeded on individual wells of the LN332E8- or LN511E8-coated plate. After incubation at 37°C for 15 minutes, non-adherent cells in some wells were removed and the remaining adherent cells were continued to be cultured. In the rest of the wells, culture was continued without removal of non-adherent cells. The cells adherent on LN211E8-coated plate were continued to be cultured after medium was added.

One day later, the phase-contrast microscopic image and the fluorescence microscopic image of each well were taken. In five fields of view in a 100-fold magnified image taken with a fluorescence microscope, the total cell number and the GFP-expressing cell (p63-positive cell) number were counted, and the percentage of p63-positive cells was calculated.

(2) Experimental Results

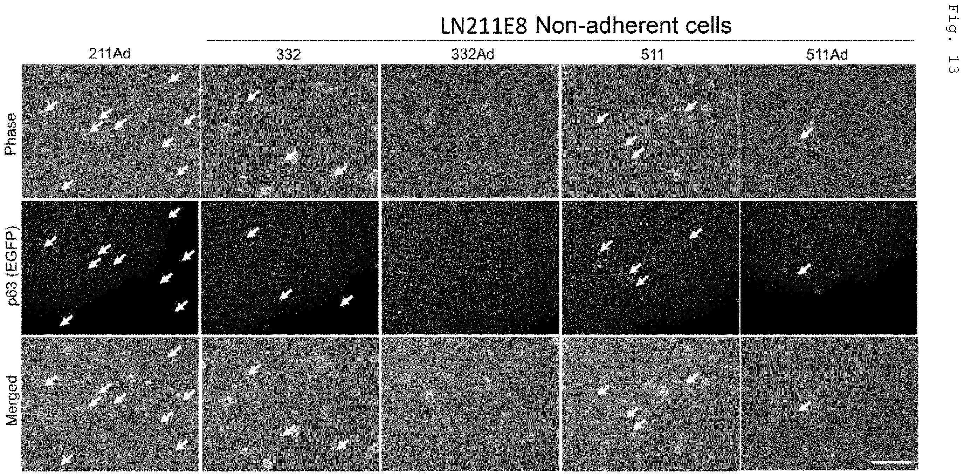

The microscopic images are shown in Fig. 13. In the figure, the top row (Phase) shows phase-contrast microscopic images, the middle row (p63 (EGFP)) shows fluorescence microscopic images, and the bottom row (Merged) shows merged images of the top and bottom panels . In the figure, "211Ad" represents a well on which the cells adherent to LN211E8 were continued to be cultured; "332" represents an LN332E8-coated well on which the cells not adherent to LN211E8 were cultured; "332Ad" represents an LN332E8-coated well on which the cells not adherent LN211E8 were cultured and subsequently depleted of non-adherent cells, "511" represents an LN511E8-coated well on which the cells not adherent to LN211E8 were cultured, and "511Ad" represents an LN511E8-coated well on which the cells not adherent LN211E8 were cultured and subsequently depleted of non-adherent cells. In the figure, each arrow represents a p63-negative cell, and the scale bar marks 100 μm. As shown in Fig. 13, p63-negative cells were hardly observed in the groups "332Ad" and "511Ad", indicating that p63-positive cells quickly adhere to LN332E8 or LN511E8.

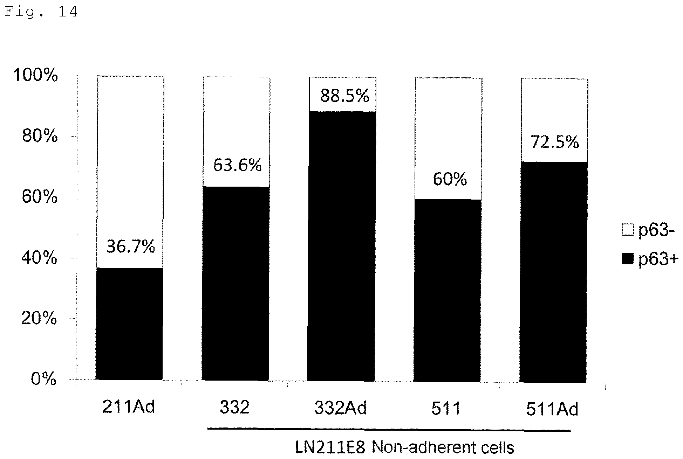

The percentage of p63-positive cells is shown in Fig. 14. Each group is as defined in Fig. 13. As is clear from Fig. 14, the percentages of p63-positive cells in the groups "332Ad" and "511Ad" were higher than those of the other groups. That is, the step of removing cells not adherent to LN332E8 or cells not adherent to LN511E8 was proved to be important for enhancing the purity of corneal epithelial cells.

The present invention is not limited to the particular embodiments and examples described above, and various modifications can be made within the scope of the appended claims. Other embodiments provided by suitably combining technical means disclosed in separate embodiments of the present invention are also within the technical scope of the present invention. All the academic publications and patent literature cited in the description are incorporated herein by reference.

**Claims**

1. A method for producing a corneal epithelial cell population for fabrication of corneal epithelial cell sheets for transplantation, the method comprising the steps of:

   (1) preparing a cell population containing corneal epithelial cells;
   (2) subjecting the cell population prepared in step (1) to magnetic-activated cell sorting and collecting CD200-negative/SSEA-4-positive cells; and
   (3) bringing the cells collected in step (2) into contact with a laminin selected from the group consisting of a laminin having an α3 chain, a laminin having an α4 chain and a laminin having an α5 chain, or an E8 fragment thereof, and collecting cells adherent thereto.

2. The method according to claim 1, wherein step (3) comprises the substeps of:

   (3-1) bringing the cells collected in step (2) into contact with a laminin having an α2 chain or an E8 fragment thereof, and collecting non-adherent cells; and
   (3-2) bringing the collected non-adherent cells into contact with a laminin selected from the group consisting of a laminin having a laminin having an α3 chain, a laminin having an α4 chain and a laminin having an α5 chain, or an E8 fragment thereof, and collecting cells adherent thereto.

3. The method according to claim 1 or 2, wherein the cell population containing corneal epithelial cells is a periocular cell population derived by induced differentiation of pluripotent stem cells.

4. The method according to any of claims 1 to 3, wherein the proportion of PAX6-positive/p63-positive cells in the corneal epithelial cell population for fabrication of corneal epithelial cell sheets for transplantation is 50% or more.

5. The method according to any of claims 1 to 4, wherein the laminin selected from the group consisting of a laminin having an α3 chain, a laminin having an α4 chain and a laminin having an α5 chain, or an E8 fragment thereof is a laminin α3β3γ2 E8 fragment, a laminin α4β1γ1 E8 fragment or a laminin α5β1γ1 E8 fragment.

6. The method according to claim 2, wherein the laminin having an α2 chain or an E8 fragment thereof is a laminin α2β1γ1 E8 fragment.

7. A method for fabricating a corneal epithelial cell sheet for transplantation, the method comprising the step of culturing the corneal epithelial cell population produced by the method according to any of claims 1 to 6.

Fig. 1

Fig. 2

(A)

(B)

Fig. 3

Fig. 4

Fig. 5

Fig. 6

Fig. 7

# Adherent cells

**(A)** *K12*

**(B)** *TP63*

**(C)** *ITGA7*

**(D)** *Vimentin*

Fig. 8

Fig. 9

## Confluent

Fig. 10

| Hoechst | PAX6 | MUC16 |
|---------|------|-------|

| Hoechst | K12 | p63 |
|---------|-----|-----|

Fig. 11

Fig. 12

Fig. 13

Fig. 14

LN211E8 Non-adherent cells

## INTERNATIONAL SEARCH REPORT

| International application No. |
|---|
| PCT/JP2017/047031 |

### A. CLASSIFICATION OF SUBJECT MATTER

Int.Cl. C12N5/071(2010.01)i, A61K35/30(2015.01)i, A61L27/38(2006.01)i,
A61P27/02(2006.01)i, C12N5/10(2006.01)i, A61K35/545(2015.01)n

According to International Patent Classification (IPC) or to both national classification and IPC

### B. FIELDS SEARCHED

Minimum documentation searched (classification system followed by classification symbols)
Int.Cl. C12N5/071, A61K35/30, A61L27/38, A61P27/02, C12N5/10, A61K35/545

| Documentation searched other than minimum documentation to the extent that such documents are included in the fields searched | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580, (JDreamIII) CAplus/MEDLINE/EMBASE/BIOSIS (STN), Japio-GPG/FX

### C. DOCUMENTS CONSIDERED TO BE RELEVANT

| Category* | Citation of document, with indication, where appropriate, of the relevant passages | Relevant to claim No. |
|---|---|---|
| A | WO 2016/114285 A1 (OSAKA UNIVERSITY) 21 July 2016, entire text & EP 3246394 A1, whole documents & KR 10-2017-0092694 A & CN 107208052 A | 1–7 |
| A | WO 2016/043168 A1 (OSAKA UNIVERSITY) 24 March 2016, entire text & EP 3196297 A1, whole documents & CN 106715685 A | 1–7 |
| A | WO 2010/134619 A1 (TOHOKU UNIVERSITY) 25 November 2010, entire text & US 2012/0142103 A1, whole documents | 1–7 |
| A | WO 2012/144582 A1 (OSAKA UNIVERSITY) 26 October 2012, entire text & US 2014/0127803 A1, whole documents & EP 2700709 A1 & CN 103492555 A | 1–7 |
| A | HAYASHI, Ryuhei, et al., "Generation of Corneal Epithelial Cells from Induced Pluripotent Stem Cells Derived from Human Dermal Fibroblast and Corneal Limbal Epithelium", PLOS ONE, 2012, vol. 7, issue 9, e45435, pp. 1–10 | 1–7 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>14 March 2018 (14.03.2018) | Date of mailing of the international search report<br>27 March 2018 (27.03.2018) |
|---|---|
| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |

Form PCT/ISA/210 (second sheet) (January 2015)

30

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HAYASHI et al.** *Nature,* 17 March 2016, vol. 531 (7594), 376-80
- **SHALOM-FEUERSTEIN, R. ; SERROR, L. ; DE LA FOREST DIVONNE, S. ; PETIT, I. ; ABERDAM, E. ; CAMARGO, L. ; DAMOUR, O. ; VIGOUROUX, C. ; SOLOMON, A. ; GAGGIOLI, C. et al.** Pluripotent stem cell model reveals essential roles for miR-450b-5p and miR-184 in embryonic corneal lineage specification. *Stem Cells,* 2012, vol. 30, 898-909
- **HEWITT, K.J. ; SHAMIS, Y. ; CARLSON, M.W. ; ABERDAM, E. ; ABERDAM, D. ; GARLICK, J.A.** Three-dimensional epithelial tissues generated from human embryonic stem cells. *Tissue Eng,* 2009, vol. 15, 3417-3426
- **HAYASHI, R. ; ISHIKAWA, Y. ; ITO, M. ; KAGEYAMA, T. ; TAKASHIBA, K. ; FUJIOKA, T. ; TSUJIKAWA, M. ; MIYOSHI, H. ; YAMATO, M. ; NAKAMURA, Y.** Generation of corneal epithelial cells from induced pluripotent stem cells derived from human dermal fibroblast and corneal limbal epithelium. *PLoS ONE,* 2012, vol. 7, e45435

- **HANSON, C. ; HARDARSON, T. ; ELLERSTROM, C. ; NORDBERG, M. ; CAISANDER, G. ; RAO, M. ; HYLLNER, J. ; STENEVI, U.** Transplantation of human embryonic stem cells onto a partially wounded human cornea in vitro. *Acta Ophthalmol. (Copenh.),* 2013, vol. 91, 127-130
- **AHMAD, S. ; STEWART, R. ; YUNG, S. ; KOLLI, S. ; ARMSTRONG, L. ; STOJKOVIC, M. ; FIGUEIREDO, F. ; LAKO, M.** Differentiation of human embryonic stem cells into corneal epithelial-like cells by in vitro replication of the corneal epithelial stem cell niche. *Stem Cells,* 2007, vol. 25, 1145-1155
- **HAYASHI R et al.** *Tissue Eng Part C Methods,* 2010, vol. 16 (4), 553-560
- **EDGAR D et al.** *J. Cell Biol.,* 1987, vol. 105, 589-598
- **HIROYUKI IDO et al.** *The Journal of Biological Chemistry,* 2007, vol. 282, 11144-11154
- **HIROYUKI IDO et al.** *The Journal of Biological Chemistry,* 2004, vol. 279, 10946-10954
- **GU J et al.** *The Journal of Biological Chemistry,* 2002, vol. 277, 19922-19928
- *Stem Cell Res.,* 31 October 2017, vol. 25, 256-265